# EUROPÄISCHE PATENTSCHRIFT

(11) **EP 3 254 066 B1**
(45) Veröffentlichungstag und Bekanntmachung des Hinweises auf die Patenterteilung: **03.04.2019**
(21) Anmeldenummer: 16702414.0
(22) Anmeldetag: 01.02.2016
(51) Int. Cl.: G01F 1/58, A61M 1/00, G01F 25/00

(54) **FLUSSMESSER UND KASSETTENMODUL FÜR EINEN FLUSSMESSER**
FLOWMETER AND CASSETTE MODULE FOR A FLOWMETER
DÉBITMÈTRE ET MODULE CASSETTE POUR UN DÉBITMÈTRE

(30) Priorität: 04.02.2015 DE 102015001406
(43) Veröffentlichungstag der Anmeldung: 13.12.2017
(73) Patentinhaber: Fresenius Medical Care Deutschland GmbH, 61352 Bad Homburg (DE)
(72) Erfinder: HEIDE, Alexander, 65817 Eppstein (DE); NIKOLIC, Dejan, 65812 Bad Soden (DE)
(74) Vertreter: Dreyhsig, Jörg
(86) Internationale Anmeldenummer: PCT/EP2016/052040
(87) Internationale Veröffentlichungsnummer: WO 2016/124527

(56) Entgegenhaltungen:
- WO-A1-00/19174
- WO-A1-2011/113838
- WO-A1-2013/164089
- GB-A- 2 003 274
- GB-A- 2 056 691
- US-A- 3 002 379
- US-A- 4 351 189
- US-A- 4 585 552

## Beschreibung

### Technisches Gebiet

Die Erfindung bezieht sich auf einen Flussmesser, insbesondere einen Differenzflussmesser zum Messen einer Differenz zwischen einem ersten Flüssigkeitsstrom und einem zweiten Flüssigkeitsstrom. Die Erfindung betrifft weiterhin ein Kassettenmodul, das Kanäle für den ersten und den zweiten Flüssigkeitsstrom aufweist, zur Verwendung in einem Differenzflussmesser.

### Hintergrund

Zur Entfernung von harnpflichtigen Substanzen und zum Flüssigkeitsentzug werden beim chronischen Nierenversagen verschiedene Verfahren zur apparativen Blutreinigung bzw. Blutbehandlung eingesetzt. Bei der Hämodialyse (HD) überwiegt der diffusive Stofftransport, bei der Hämofiltration (HF) liegt ein konvektiver Stofftransport über die Membran vor. Eine Kombination aus beiden Verfahren ist die Hämodiafiltration (HDF). Bei der Peritonealdialyse (PD) wird kein extrakorporaler Kreislauf benötigt und das Peritoneum als Kontaktmembran ausgenutzt.

Wegen der großen Austauschmengen besteht bei den genannten Verfahren, sowie auch bei der kontinuierlichen arterio-venösen HF, der kontinuierlichen venovenösen HF und der Plasmafiltration (PF) die Notwendigkeit der exakten Bilanzierung von entzogener Flüssigkeit einerseits zur zugeführten Flüssigkeit andererseits und der zu ultrafiltrierenden Menge über die gesamte Behandlungszeit. Zum Stand der Technik gehören gravimetrische und volumetrische Bilanziersysteme.

Darüber hinaus sind auch Verfahren bekannt, die die Flüssigkeitsströme der in den Dialysator einfließenden Flüssigkeiten und der aus dem Dialysator ausfließenden Flüssigkeiten kontinuierlich messen und gegeneinander bilanzieren. Hierzu werden Durchflussmesssensoren oder Flussmesser verschiedener Ausgestaltungen verwendet.

Magnetische Flussmesser, die auch als elektromagnetische Flussmesser oder induktive Flussmesser bezeichnet werden, beruhen auf der Messung der Strömungsgeschwindigkeit einer leitfähigen Flüssigkeit durch ein bekanntes oder kontrolliertes magnetisches Feld durch Messung der induzierten elektrischen Spannung. Bei bekanntem Strömungsquerschnitt kann von der Strömungsgeschwindigkeit auf die Flussrate oder den Volumenstrom geschlossen werden, was von dem Begriff des Flüssigkeitsstromes im Folgenden umfasst sein soll. Eine elektrische Spannung entsteht in einem durchströmten Magnetfeld durch eine Ladungstrennung der in einer leitfähigen Flüssigkeit vorliegenden Ionen, die als induzierte Spannung gemessen werden kann. Die Spannungsmessung erfolgt typischerweise, indem die induzierte Spannung an einem Paar von Elektroden, die in elektrischem Kontakt zu der leitfähigen Flüssigkeit stehen, oder die kapazitiv mit der Flüssigkeit gekoppelt sind, abgeleitet wird. Diese Spannung ist proportional zu der Strömungsgeschwindigkeit und abhängig von der Magnetfeldstärke. Die Ladungstrennung erfolgt in senkrechter Richtung zu der Flussrichtung sowie zu der Richtung des Magnetfeldes. Das Magnetfeld eines magnetischen Flussmessers ist daher vorzugsweise senkrecht zur Flussrichtung in dem entsprechenden Flüssigkeitskanal angeordnet und das Elektrodenpaar zum Ableiten der induzierten elektrischen Spannung ist vorzugsweise senkrecht sowohl zu dem Magnetfeld als auch zur Flussrichtung in dem Flüssigkeitskanal angeordnet.

Ein typischer elektromagnetischer Flussmesser ist aus einer nicht-magnetischen und nicht magnetisierbaren Röhre aufgebaut, die auf der Innenseite mit elektrisch isolierendem Material ausgekleidet ist.

Typischerweise wird das magnetische Feld durch eine oder mehrere außerhalb der flüssigkeitsdurchströmten Röhre angeordnete Spulen erzeugt. Die durch den Flüssigkeitsstrom induzierte elektrische Spannung wird typischerweise durch einen Spannungsmesser bestimmt und das Ergebnis der Spannungsmessung wird einer Auswerteeinheit zugeführt zur Bestimmung des Flüssigkeitsstromes, d.h. der Flussrate oder des Volumenstroms, basierend auf der gemessenen Spannung.

Wird ein elektromagnetischer Flussmesser als Differenzflussmesser zum Messen einer Flussdifferenz zwischen einem ersten und einem zweiten fluidführenden Kanal ausgebildet, so durchdringt vorteilhaft ein gemeinsames Magnetfeld den ersten und den zweiten fluidführenden Kanal.

Entsprechen der erste und der zweite fluidführende Kanal einander hinsichtlich ihrer geometrischen Abmessungen, so gibt die Spannungsdifferenz zwischen einem an dem ersten fluidführenden Kanal angeordneten ersten Elektrodenpaar und einem an dem zweiten fluidführenden Kanal angeordneten zweiten Elektrodenpaar unmittelbar die Differenz zwischen dem Fluss in dem ersten Kanal und dem Fluss in dem zweiten Kanal an. Werden das erste und das zweite Elektrodenpaar in Reihe geschaltet, so kann diese Spannungsdifferenz unmittelbar abgegriffen werden.

Ein elektromagnetischer Differenzflussmesser ist vorteilhaft aus dem einen Kassettenmodul, in dem die fluidführenden Kanäle ausgebildet sind, die jeweils ein Elektrodenpaar aufweisen, einem Elektromagneten oder Permanentmagneten zum Erzeugen eines Magnetfeldes zwischen den Elektrodenpaaren und einer Auswerteeinheit und zum Auswerten der Spannungen oder der Differenzspannung zwischen den Elektrodenpaaren aufgebaut.

Differenzflussmesser nach dem elektromagnetischen Prinzip besitzen eine hohe Empfindlichkeit gegenüber einer Lageänderung (in Bezug auf das Magnetfeld) der Fluidführenden Kanäle.

Aus der WO 2011/113838 A1 ist eine Kassette mit einem ersten und einem zweiten Flüssigkeitsstrom in einem Dialysesystem bekannt, wobei die Kassette einen Sensor als Vorrichtung zur Bestimmung einer Differenz des ersten und des zweiten Flüssigkeitsstroms aufweist und wobei der Sensor einen ersten Kanal für den ersten Flüssigkeitsstrom und einen zweiten Kanal für den zweiten Flüssigkeitsstrom aufweist.

Aus der US 4 585 552 A ist Dialyseausrüstung bekannt, aufweisend einen Dialysator, ein System zum Messen einer Differenz in der Flussrate zwischen einem ersten und einem zweiten Flüssigkeitsstrom, wobei der erste Flüssigkeitsstrom das Strömen von frischer Dialyselösung zum Dialysator umfasst und wobei der zweite Flüssigkeitsstrom das Strömen von gebrauchter Dialyselösung vom Dialysator hinweg umfasst, wobei das System einen ersten Kanal zur Aufnahme und Durchleitung des ersten Flüssigkeitsstroms, einen zweiten Kanal zur Aufnahme und Durchleitung des zweiten Flüssigkeitsstroms und Messmittel zur Messung der Differenz zwischen den Flussraten des ersten und des zweiten Flüssigkeitsstroms in dem ersten und dem zweiten Kanal aufweist.

Es ist daher die Aufgabe der vorliegenden Erfindung, einen verbesserten Differenzflussmesser zur Verfügung zu stellen.

### Zusammenfassung

Diese Aufgabe wird gelöst durch ein Kassettenmodul nach Anspruch 1, sowie durch einen Differenzflussmesser nach Anspruch 3. Vorteilhafte Weiterbildungen sind in den abhängigen Ansprüchen angegeben.

Im Einklang mit der Lehre der vorliegenden Erfindung wird ein Kassettenmodul für einen Differenzflussmesser bereitgestellt. In dem Kassettenmodul sind ein erster und eine zweiter Kanal ausgebildet, die im Betrieb des Differenzflussmessers fluidführend sind und die im Betrieb des Differenzflussmessers jeweils von einem Magnetfeld durchdrungen sind. An dem ersten und an dem zweiten Kanal sind jeweils ein Elektrodenpaar angeordnet, d.h. ein erstes Elektrodenpaar an dem ersten Kanal und ein zweites Elektrodenpaar an dem zweiten Kanal. Durch einen Vergleich des Signals am ersten und am zweiten Elektrodenpaar ist eine Flussdifferenz zwischen dem ersten und dem zweiten fluidführenden Kanal bestimmbar, etwa eine Differenz zwischen dem Volumenstrom zwischen dem im ersten Kanal und dem im zweiten Kanal strömenden Fluid, basierend auf einer Differenz der Flussgeschwindigkeit im ersten und im zweiten Kanal und der Kenntnis der jeweiligen Kanalgeometrie. Der erste im Betrieb fluidführende Kanal weist einen ersten Abschnitt, in dem das Fluid in eine vorbestimmte Richtung strömt und einen weiteren Abschnitt auf, vorzugsweise einen rückführenden Abschnitt, d.h. einen Abschnitt, in dem das Fluid im Wesentlichen in eine der vorbestimmten Richtung entgegengesetzte Richtung strömt. An dem rückführenden Abschnitt ist ein weiteres Elektrodenpaar angeordnet. Durch einen Vergleich des Signals an dem ersten und an dem weiteren Elektrodenpaar kann eine Änderung der Messbedingungen festgestellt werden.

In einer vorteilhaften Ausführungsform des Kassettenmoduls bildet auch der zweite im Betrieb fluidführende Kanal einen im Betrieb von dem Magnetfeld durchdrungenen weiteren Abschnitt aus, vorzugsweise einen rückführenden Abschnitt, d.h. der zweite Kanal bildet einen ersten und den rückführenden Abschnitt aus, wobei die Strömungsrichtung im ersten und im weiteren Abschnitt im wesentlichen jeweils in eine entgegengesetzte Richtung weisen. An dem zweiten Abschnitt ist ein viertes Elektrodenpaar angeordnet. So kann durch einen Vergleich der Spannung an dem zweiten Elektrodenpaar und der Spannung an dem vierten Elektrodenpaar eine Änderung der Messbedingungen festgestellt werden.

Auf diese Weise ist eine zusätzliche Überprüfungsmöglichkeit der Messbedingungen gegeben.

Im Einklang mit der Lehre der vorliegenden Erfindung wird weiterhin ein Differenzflussmesser mit einem oben beschriebenen Kassettenmodul bereitgestellt. Der Differenzflussmesser weist eine mit dem ersten, dem zweiten und dem weiteren Elektrodenpaar verbundene Auswerteeinheit auf. Die Auswerteeinheit ist so konfiguriert, dass durch einen Vergleich des Signals am ersten und am zweiten Elektrodenpaar eine Flussdifferenz zwischen dem ersten und dem zweiten fluidführenden Kanal bestimmbar ist, und weiterhin so konfiguriert ist, dass durch einen Vergleich des Signals an dem ersten und an dem weiteren Elektrodenpaar eine Änderung der Messbedingungen feststellbar ist. In einer Ausführungsform weist der Differenzflussmesser einen Permanent- oder Elektromagneten zum Erzeugen des Magnetfeldes auf.

In einer Weiterbildung des Differenzflussmessers ist die Auswerteeinheit so konfiguriert, dass durch einen Vergleich des Signals an dem ersten und an dem weiteren Elektrodenpaar eine Lageänderung des Kassettenmoduls im Magnetfeld feststellbar ist.

Mit anderen Worten wird eine Änderung der Messbedingungen dahingehend interpretiert, dass sie auf eine Lageänderung des Kassettenmoduls im Magnetfeld zurückgeht.

In einer vorteilhaften Weiterbildung des Differenzflussmessers ist die Auswerteeinheit so konfiguriert, dass durch einen Vergleich des Signals an dem zweiten und an dem vierten Elektrodenpaar eine Änderung der Messbedingungen feststellbar ist.

Auf diese Weise ist eine zusätzliche Überprüfungsmöglichkeit der Messbedingungen gegeben.

In einer Ausführungsform des Differenzflussmessers ist die Auswerteeinheit so konfiguriert, dass durch einen Vergleich des Signals an dem zweiten und an dem vierten Elektrodenpaar eine Lageänderung des Kassettenmoduls im Magnetfeld feststellbar ist.

Somit wird eine Änderung der Messbedingungen dahingehen interpretiert, dass sie auf eine Lageänderung des Kassettenmoduls im Magnetfeld zurückgeht.

In einer vorteilhaften Ausführungsform des Differenzflussmessers weist der zweite fluidführende Kanal einen ersten und den rückführenden Abschnitt auf und der erste und der rückführende Abschnitt sind in Bezug auf die Richtung des Magnetfelds nebeneinander angeordnet.

So kann bei einer Inhomogenität des Magnetfelds eine Lageänderung des Kassettenmoduls mit einer großen Sensitivität erkannt werden.

In einer vorteilhaften Weiterbildung des Differenzflussmessers sind der erste und der zweite fluidführende Kanal in Bezug auf die Richtung des Magnetfelds hintereinander angeordnet.

Auf diese Weise kann ein Differenzflussmesser so ausgestaltet werden, dass nur eine geringe Empfindlichkeit gegenüber Lageänderungen des Kassettenmoduls im Magnetfeld gegeben ist.

In einer weiteren vorteilhaften Weiterbildung des Differenzflussmessers weist der erste fluidführende Kanal einen ersten und den rückführenden Abschnitt auf und der erste und der rückführende Abschnitt sind in Bezug auf die Richtung des Magnetfelds nebeneinander angeordnet.

Auf diese Weise kann bei einer Inhomogenität des Magnetfelds eine Lageänderung des Kassettenmoduls mit einer großen Sensitivität erkannt werden.

In einer vorteilhaften Weiterbildung des Differenzflussmessers ist die Auswerteeinheit angepasst, als Antwort auf eine Feststellung der Änderung der Messbedingungen eine Rekalibrierung zu initiieren und/oder durchzuführen.

Auf diese Weise kann durch eine Rekalibrierung die Messgenauigkeit wieder hergestellt werden.

In einer weiteren Ausführungsform des Differenzflussmessers ist eine Kurzschlussleitung und eine Absperrorgan zum Kurzschließen des ersten und des zweiten Kanals vorgesehen, und das Absperrorgan ist derart ansteuerbar, dass bei der Rekalibrierung der erste und der zweite Kanal kurzgeschlossen werden.

### Kurzbeschreibung der Zeichnungen

Es zeigen:
**Fig. 1** einen typischen Aufbau eines Differenzflussmessers mit einem Kassettenmodul, (Stand der Technik).
**Fig. 2a** die geometrischen Lagebeziehungen in dem in Fig.1 dargestellten Kassettenmodul in einer Idealposition.
**Fig. 2b** die geometrischen Lagebeziehungen in dem in Fig. 1 dargestellten Kassettenmodul in einer von der Idealposition abweichenden Position.
**Fig. 2c** die geometrischen Lagebeziehungen in dem in Fig. 1 dargestellten Kassettenmodul in einer weiteren von der Idealposition abweichenden Position.
**Fig. 2d** die geometrischen Lagebeziehungen in dem in Fig. 1 dargestellten Kassettenmodul in einer dritten von der Idealposition abweichenden Position.
**Fig. 3a** eine Anordnung von fluidführenden Kanälen eines Kassettenmoduls in einem Magnetfeld.
**Fig. 3b** eine weitere Anordnung von fluidführenden Kanälen eines Kassettenmoduls in einem Magnetfeld.
**Fig. 4a** eine Ausgestaltung eines Differenzflussmessers im Einklang mit der vorliegenden Lehre.
**Fig. 4b** eine weitere Ausgestaltung eines Differenzflussmessers im Einklang mit der vorliegenden Lehre.
**Fig. 5** eine Anordnung von fluidführenden Kanälen eines Kassettenmoduls im Magnetfeld im Einklang mit der vorliegenden Lehre.
**Fig. 6a** eine Ausgestaltung eines Differenzflussmessers mit einer Kalibriereinheit in einem ersten Betriebsmodus.
**Fig. 6b** eine Ausgestaltung eines Differenzflussmessers mit einer Kalibriereinheit in einem weiteren Betriebsmodus.

### Detaillierte Beschreibung eines Ausführungsbeispiels

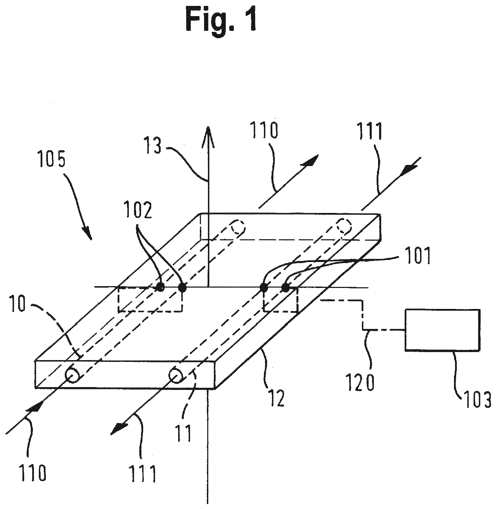

Fig. 1 zeigt den typischen Aufbau eines elektromagnetischen Differenzflussmessers 105 zum Bestimmen einer Flüssigkeitsbilanz. Der Differenzflussmesser 105 weist ein Kassettenmodul 12 auf, in dem die fluidführenden Kanäle 10 und 11 ausgebildet sind, zwischen denen Flüssigkeit bilanziert werden soll. Die fluidführenden Kanäle 10 und 11 sind im Gebrauch des Differenzflussmessers typischerweise an einen Flüssigkeitskreislauf angeschlossen, in dem eine Flüssigkeitsbilanz zu bestimmen ist, etwa in dem Dialysierflüssigkeitskreislauf einer Dialysevorrichtung.
Das Kassettenmodul 12 ist von einem durch die Magnetfeldlinie angedeuteten Magnetfeld 13 durchdrungen, das von einem Elektro- oder Permanentmagneten (nicht dargestellt) erzeugt wird.

Der erste fluidführende Kanal 10 wird in der angedeuteten Strömungsrichtung 110 durchströmt, entgegengesetzt zur Strömungsrichtung 111 in dem zweiten fluidführenden Kanal 11.

Die in der Flüssigkeit in dem Kanälen 10 und 11 vorhandenen Ionen unterliegen durch die Strömung senkrecht zum Magnetfeld 13 einer Ladungstrennung, die als Potentialdifferenz an den jeweils an den flüssigkeitsführenden Kanälen angeordneten ersten 102 und zweiten 101 Elektrodenpaar abgeleitet werden kann.

Das erste Elektrodenpaar 102 und das zweite Elektrodenpaar 101 sind über Messleitungen 120 mit der Auswerteeinheit 103 verbunden.

Durch Vergleich der an dem ersten Elektrodenpaar 102 abgeleiteten Spannung und der am zweiten Elektrodenpaar 101 abgeleiteten Spannung wird in der Auswerteeinheit 103 eine Flüssigkeitsbilanz oder ein Differenzfluss zwischen dem ersten 10 und dem zweiten 11 fluidführenden Kanal bestimmt, etwa als Differenz der Volumenströme im ersten 10 und im zweiten 11 fluidführenden Kanal.

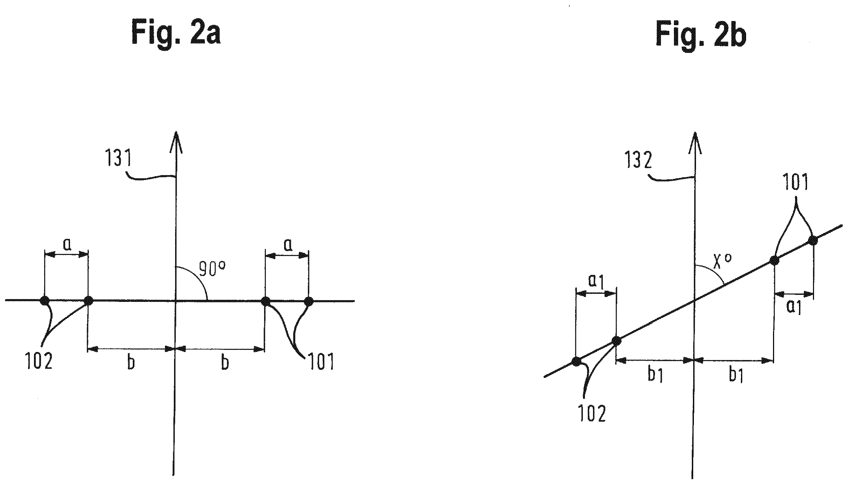

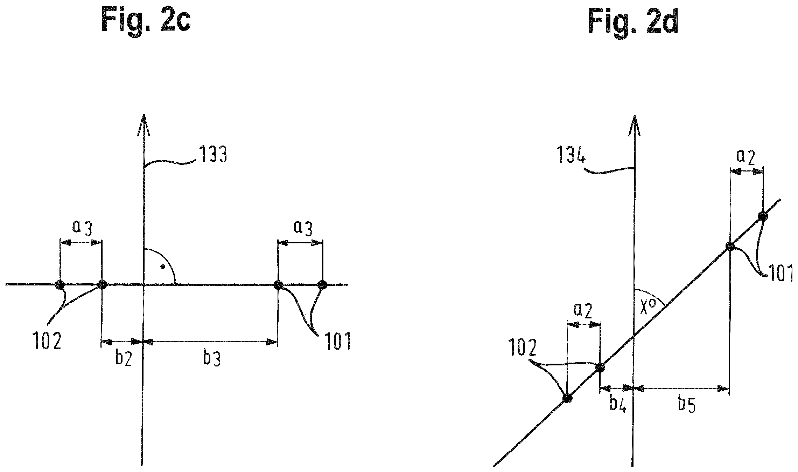

Fig. 2a zeigt die Anordnung der Elektrodenpaare 102, 101 im Magnetfeld sowie die geometrischen Abmessungen in dem in der Figur 1 dargestellten Differenzflussmesser in einer Idealposition. Ein Richtungspfeil deutet die Richtung 131 des Magnetfelds in dem Differenzflussmesser an, sowie eine Symmetrielinie des Magnetfelds an. Die Elektrodenpaare 102 und 101 liegen auf einer gedachten Linie senkrecht zur Richtung 131 des Magnetfelds. Mit 'a' ist jeweils der - übereinstimmende - Abstand der Elektroden des ersten 102 und des zweiten 101 Elektrodenpaars bezeichnet. Mit 'b' ist jeweils der -übereinstimmende - Abstand der der Mitte zugewandten Elektrode des jeweiligen Elektrodenpaars 102, 101 zu der gedachten Mittellinie zwischen den Elektrodenpaaren bezeichnet. Die in Figur 2 a dargestellte Konfiguration ist eine ideale Konfiguration, in dem Sinn, das die Abstand 'a' zwischen den Elektroden der Elektrodenpaare 102, 101 jeweils übereinstimmt, und das die Elektrodenpaare 102, 101 jeweils den gleichen Abstand 'b' zur Symmetrielinie des Magnetfelds haben. So kann über einen Vergleich der an dem ersten Elektrodenpaar 102 abgeleiteten Spannung und der am zweiten Elektrodenpaar 101 abgeleiteten Spannung, beispielsweise durch Differenzbildung zwischen der am ersten und am zweiten Elektrodenpaar abgeleiteten Spannung auf die Strömungsdifferenz zwischen der Strömung im ersten Kanal und der Strömung im zweiten Kanal geschlossen werden, beispielsweise einer Differenz zwischen dem Volumenstroms im ersten und dem im zweiten Kanal. So entspricht bei der in Figur 2a dargestellten idealen Konfiguration eine Spannungsdifferenz null zwischen der Spannung am ersten 102 und der Spannung am zweiten Elektrodenpaar 101 einer Strömungsdifferenz null. Eine Beziehung zwischen den an den Elektrodenpaaren 102, 101 abgeleiteten Spannungen und den jeweiligen Volumenströmen kann mit Hilfe von Kalibrationsmessungen hergestellt werden und ist in der Auswerteeinheit 103 abgelegt.

Fig. 2b zeigt die Anordnung der Elektrodenpaare 102, 101 im Magnetfeld sowie die geometrischen Abmessungen in dem in der Figur 1 dargestellten Differenzflussmesser in einer von der Idealposition abweichenden Position. Ein Richtungspfeil deutet die Richtung 132 des Magnetfelds in dem Differenzflussmesser an, die von der Symmetrielinie des Magnetfelds abweicht. Die Elektrodenpaare 102 und 101 liegen auf einer gedachten Linie in einem Winkel von x° zur Richtung 132 des Magnetfelds. Mit 'a1' ist jeweils die - übereinstimmende - Projektion des Abstands der Elektroden des ersten 102 und des zweiten 101 Elektrodenpaars auf eine Linie senkrecht zur Richtung 132 des Magnetfelds bezeichnet. Mit 'b1' ist jeweils der -übereinstimmende - Projektion des Abstands der der Mitte zugewandten Elektrode des jeweiligen Elektrodenpaars 102, 101 zu eine Linie senkrecht zur Richtung des Magnetfelds bezeichnet. Die in Figur 2 b dargestellte Konfiguration weicht von der idealen Konfiguration ab, in dem Sinn, das die Projektion des Abstands 'a1' gegenüber dem Abstand in Figur 2a mit 'a' bezeichneten Abstand zwischen den Elektroden der Elektrodenpaare 102, 101 abweicht, und das die Projektionen 'b1' des Abstands der Elektrodenpaare 102, 101 von dem in Figur 2a mit 'b' bezeichneten Abstand abweichen. Die Änderung des Abstands 'a' zu 'a1' führt bei ansonsten identischen Flüssen und identischem Betrag des Magnetfelds zu einer Änderung der an den Elektrodenpaaren 102, 101 abfallenden Spannungen. Fall das Magnetfeld Inhomogenitäten aufweist, führt eine Änderung des Abstands 'b' zu 'b1' zu einer betragsmäßigen Änderung des Magnetfelds am Ort des ersten Elektronenpaars 102 und am Ort des zweiten Elektronenpaars 101. Somit Ändern sich die am ersten 102 und am zweiten 101 Elektronenpaar abgeleiteten Spannungen bei unveränderten Volumenströmen, und die auf den abgeleiteten Spannungen basierenden Messergebnisse.

Fig. 2c zeigt die Anordnung der Elektrodenpaare 102, 101 im Magnetfeld sowie die geometrischen Abmessungen in dem in der Figur 1 dargestellten Differenzflussmesser in einer weiteren von der Idealposition abweichenden Position. Ein Richtungspfeil deutet die Richtung 133 des Magnetfelds in dem Differenzflussmesser an, sowie eine Symmetrielinie des Magnetfelds an. Die Elektrodenpaare 102 und 101 liegen auf einer gedachten Linie senkrecht zur Richtung 133 des Magnetfelds. Mit ,a3' ist jeweils der - übereinstimmende - Abstand der Elektroden des ersten 102 und des zweiten 101 Elektrodenpaars bezeichnet. Mit 'b2' und 'b3' sind jeweils die unterschiedlichen Abstände der der Mitte zugewandten Elektrode des jeweiligen Elektrodenpaars 102, 101 zu der gedachten Mittellinie zwischen den Elektrodenpaaren bezeichnet. Die in Figur 2c dargestellte Konfiguration weicht insofern von der idealen Konfiguration ab, als die Elektrodenpaare 102 und 101 nicht symmetrisch im Magnetfeld angeordnet sind. Weist das Magnetfeld Inhomogenitäten oder eine Asymmetrie auf, so ändern sich die an dem ersten 102 und zweiten 101 Elektrodenpaar abgeleiteten Spannungen, bei ansonsten unveränderten Bedingungen, etwa unveränderten Flüssigkeitsflüssen.

Fig. 2d zeigt die Anordnung der Elektrodenpaare 102, 101 im Magnetfeld sowie die geometrischen Abmessungen in dem in der Figur 1 dargestellten Differenzflussmesser in einer dritten von der Idealposition abweichenden Position. Ein Richtungspfeil deutet die Richtung 134 des Magnetfelds in dem Differenzflussmesser an, die von der Symmetrielinie des Magnetfelds abweicht. Die Elektrodenpaare 102 und 101 liegen auf einer gedachten Linie in einem Winkel x° zur Richtung 134 des Magnetfelds. Mit 'a2' ist jeweils die - übereinstimmende - Projektion des Abstand der Elektroden des ersten 102 und des zweiten 101 Elektrodenpaars auf eine Linie senkrecht zur Richtung 134 des Magnetfelds bezeichnet. Mit 'b4' und 'b5' sind jeweils unterschiedliche Projektionen des Abstands der der Mitte zugewandten Elektrode des jeweiligen Elektrodenpaars 102, 101 zu eine Linie senkrecht zur Richtung des Magnetfelds bezeichnet. Die in Figur 2d dargestellte Konfiguration weicht von der idealen Konfiguration ab, in dem Sinn, das die Projektion des Abstands ,a2' gegenüber dem Abstand in Figur 2a mit 'a' bezeichneten Abstand zwischen den Elektroden der Elektrodenpaare 102, 101 abweicht, und das die Elektrodenpaare 102, 101 nicht symmetrisch im Magnetfeld angeordnet sind. Dies führt sowohl betragsmäßig zu einer Änderung der an den Elektrodenpaaren 102, 101 abfallenden Spannungen, als auch zu unterschiedlich großen Spannungen, an den Elektrodenpaaren 102 und 101, wenn die Volumenströme im ersten Kanal und im zweiten Kanal gleich sind - bezogen auf die Darstellung der Figur 1.

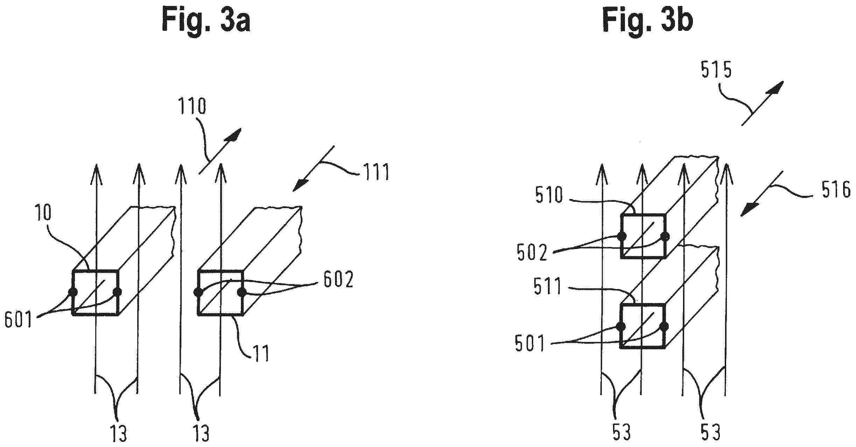

Figur 3a zeigt eine räumliche Darstellung einer Anordnung von fluidführenden Kanälen 11, 10 eines Kassettenmoduls in einem elektromagnetischen Differenzflussmesser. Der Differenzflussmesser entspricht in seiner Funktion im Wesentlichen dem im Zusammenhang mit der Figur 1 beschriebenen Differenzflussmesser, auf die Bezug genommen wird an Stelle einer Wiederholung. Die Anordnung der fluidführenden Kanäle 11, 10 in Bezug auf die Richtung des Magnetfelds 13 entspricht der in der Figur 1 dargestellten Anordnung, d.h. der erste 10 und der zweite 11 fluidführende Kanal sind in Bezug auf die Richtung des Magnetfelds nebeneinander angeordnet. Der erste fluidführende Kanal 10 wird in einer Strömungsrichtung 110 im Wesentlichen entgegengesetzt zur Strömungsrichtung 111 im zweiten fluidführenden Kanal 11 durchströmt. Elektrodenpaare 601, 602, sind an dem ersten 10 und am zweiten 11 fluidführenden Kanal angeordnet. Hinsichtlich der Lage der Elektroden zum Magnetfeld 13 und zur Strömungsrichtung 110, 111 in den Kanälen entspricht dies im Wesentlichen der in der Figur 1 dargestellten Situation.

Figur 3b zeigt eine räumliche Darstellung einer Anordnung von fluidführenden Kanälen 511, 510 eines Kassettenmoduls in Bezug auf ein Magnetfeld 53 in einem elektromagnetischen Differenzflussmesser. Hinsichtlich der Funktion entspricht der Differenzflussmesser im Wesentlichen dem im Zusammenhang mit der Figur 1 beschriebenen Differenzflussmesser, auf die Bezug genommen wird an Stelle einer Wiederholung. Die in Figur 3b dargestellte Anordnung der fluidführenden Kanäle 511, 510 in Bezug auf die Richtung des Magnetfelds 53 unterscheidet sich von der Anordnung der Figuren 1 und 3a, d.h. der erste 510 und der zweite 511 fluidführende Kanal sind in Bezug auf die Richtung des Magnetfelds hintereinander angeordnet. Bei dieser Anordnung ist nur eine geringe Empfindlichkeit des Differenzflusssensors gegeben, wenn sich die Lage der fluidführenden Kanäle 510, 511 im Magnetfeld ändert. Der erste fluidführende Kanal 510 wird in einer Strömungsrichtung 515 im wesentlichen entgegengesetzt zur Strömungsrichtung 516 im zweiten fluidführenden Kanal 511 durchströmt. Elektrodenpaare 501, 502, sind an dem ersten 510 und am zweiten 511 fluidführenden Kanal angeordnet, die Verbindungslinien zwischen den Elektroden der Elektrodenpaare 501, 502 sind jeweils im Wesentlichen senkrecht zur Richtung des Magnetfelds 53, sowie zur Strömungsrichtung 515 und zur Strömungsrichtung 516 angeordnet.

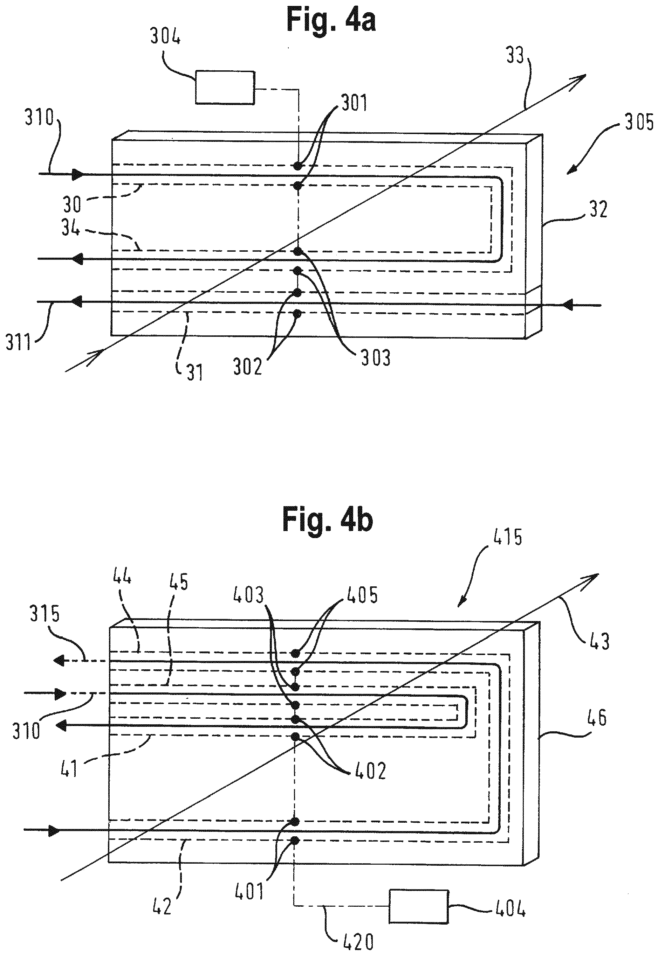

Fig. 4a zeigt den Aufbau eines elektromagnetischen Differenzflussmessers 305 zum Bestimmen einer Flüssigkeitsbilanz, in einem Ausführungsbeispiel im Einklang mit der Lehre der vorliegenden Erfindung. Der Differenzflussmesser 305 weist ein Kassettenmodul 32 auf, in dem ein erster fluidführender Kanal 30, sowie eine zweiter fluidführender Kanal 31 ausgebildet sind, zwischen denen Flüssigkeit bilanziert werden soll. Der erste fluidführende Kanal weist einen ersten Abschnitt sowie einen rückführenden Abschnitt 34 auf, d.h. in dem ersten Abschnitt strömt das zu bilanzierende Fluid in eine vorbestimmte Richtung und in dem rückführenden Abschnitt strömt das Fluid im Wesentlichen in eine der vorbestimmten Richtung entgegengesetzten Richtung.

Die fluidführenden Kanäle 30 und 31 sind im Gebrauch des Differenzflussmessers typischerweise an einen Flüssigkeitskreislauf angeschlossen, in dem eine Flüssigkeitsbilanz bestimmen werden soll, etwa in dem Dialysierflüssigkeitskreislauf einer Dialysevorrichtung.

Das Kassettenmodul 32 ist von einem durch die Magnetfeldlinie angedeuteten Magnetfeld 33 durchdrungen, das von einem Elektro- oder Permanentmagneten (nicht dargestellt) erzeugt wird.

Die Strömungsrichtung im ersten und im rückführenden Abschnitt 34 ist jeweils senkrecht zur Richtung des Magnetfeldes 33.

Im ersten Abschnitt wird der erste fluidführende Kanal 30 in der angedeuteten Strömungsrichtung 310 durchströmt, entgegengesetzt zur Strömungsrichtung 311 in dem zweiten fluidführenden Kanal 31.

Die in der Flüssigkeit in dem ersten Kanal 30 vorhandenen Ionen unterliegen durch die Strömung senkrecht zum Magnetfeld 33 einer Ladungstrennung. Diese kann als Potentialdifferenz an jeweils an dem ersten und dem rückführenden Abschnitt des ersten Kanals angeordneten ersten 301 und weiterem 303 Elektrodenpaar abgeleitet werden.

Die Ladungstrennung der im zweiten Kanal 31 vorhandenen Ionen kann als Potentialdifferenz an einem zweiten Elektrodenpaar 302 abgeleitet werden.

Das erste Elektrodenpaar 301, das zweite Elektrodenpaar 302, sowie das weitere Elektrodenpaar 303 sind über Messleitungen 320 mit der Auswerteeinheit 304 verbunden.

Die Auswerteeinheit ist konfiguriert, um einerseits eine Flüssigkeitsbilanz zwischen dem Volumenstrom im ersten 30 und im zweiten 31 fluidführenden Kanal zu bestimmen und andererseits eine Änderung der Messbedingungen, insbesondere eine Lageänderung des Kassettenmoduls 32 im Magnetfeld 33 festzustellen.

Durch Vergleich der an dem ersten Elektrodenpaar 301 abgeleiteten Spannung und der am zweiten Elektrodenpaar 302 abgeleiteten Spannung ist in der Auswerteeinheit 304 eine Flüssigkeitsbilanz oder ein Differenzfluss zwischen dem ersten 30 und dem zweiten 31 fluidführenden Kanal bestimmbar, etwa als Differenz zwischen dem Volumenstrom im ersten fluidführenden Kanal 30 und dem im zweiten fluidführenden Kanal 31.

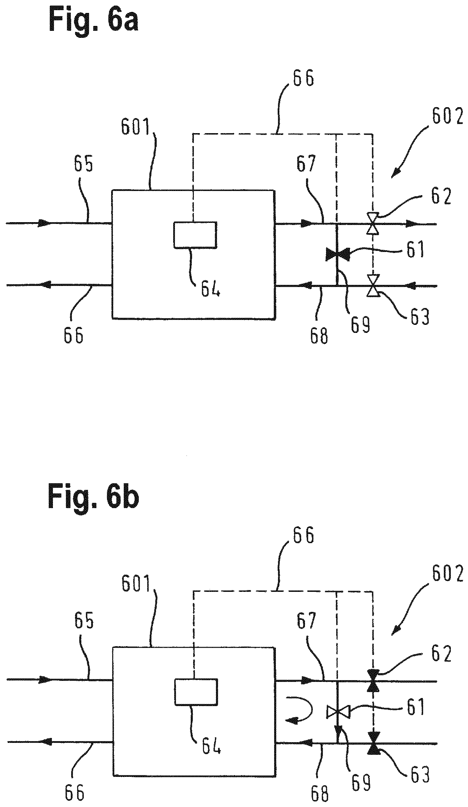

Wenn durch Ansteuern einer Kalibrationsvorrichtung gleiche Volumenströme in ersten fluidführenden Kanal 30 und im zweiten fluidführenden Kanal 31 vorherrschen, so kann durch Vergleich der am ersten Elektrodenpaar 301 abgeleiteten Spannung mit der am zweiten Elektrodenpaar 302 abgeleiteten Spannung ein Nullpunkt der Flüssigkeitsbilanz bestimmt und als Kalibrationswert hinterlegt werden. Eine entsprechende Kalibrationsvorrichtung ist im Zusammenhang mit den Figuren 6 a und 6 b beschrieben.

Durch Vergleich der an dem ersten Elektrodenpaar 301 abgeleiteten Spannung und der am weiteren Elektrodenpaar 303 abgeleiteten Spannung ist in der Auswerteeinheit 304 eine Änderung der Messbedingungen bestimmbar, insbesondere eine Lageänderung des Kassettenmoduls 32 im Magnetfeld.

Wird eine Änderung der Messbedingungen festgestellt, so wird vorteilhaft eine Kalibrierung initiiert und wie oben beschrieben durchgeführt.

Fig. 4b zeigt den Aufbau eines elektromagnetischen Differenzflussmessers 415 zum Bestimmen einer Flüssigkeitsbilanz, in einem weiteren Ausführungsbeispiel im Einklang mit der Lehre der vorliegenden Erfindung. Der Differenzflussmesser 415 weist ein Kassettenmodul 46 auf, in dem ein erster im Betrieb fluidführender Kanal 45, sowie ein zweiter im Betrieb fluidführenden Kanal 44 ausgebildet sind, zwischen denen eine Flüssigkeitsbilanz bestimmt werden soll. Der erste fluidführende Kanal 45 sowie der zweite fluidführende Kanal 44 weisen jeweils einen ersten Abschnitt sowie einen rückführenden Abschnitt auf, d.h. der erste Kanal 45 weist einen rückführenden Abschnitt 41 und der zweite Kanal 44 weist einen rückführenden Abschnitt 42 auf. Sowohl in dem ersten 45 als auch im zweiten 44 Kanal strömt in dem ersten Abschnitt das zu bilanzierende Fluid jeweils in eine bestimmte Richtung und in dem rückführenden Abschnitt strömt das Fluid im Wesentlichen in eine der dieser Richtung entgegengesetzte Richtung.

Die fluidführenden Kanäle 45 und 44 sind im Gebrauch des Differenzflussmessers typischerweise an einen Flüssigkeitskreislauf angeschlossen, in dem eine Flüssigkeitsbilanz bestimmen werden soll, etwa in dem Dialysierflüssigkeitskreislauf einer Dialysevorrichtung.

Das das Kassettenmodul 46 durchdringende Magnetfeld 43 wird von einem Elektro- oder Permanentmagneten (nicht dargestellt) erzeugt.

Für den ersten 45 und den zweiten 44 Kanal gilt, dass die Strömungsrichtung im ersten und im rückführenden Abschnitt 41, 42 jeweils im Wesentlichen senkrecht zur Richtung des Magnetfeldes liegt.

Im ersten Abschnitt 41 wird der erste fluidführende Kanal 45 in der angedeuteten Strömungsrichtung 310 durchströmt, entgegengesetzt zur Strömungsrichtung 315 in dem ersten Abschnitt des zweiten fluidführenden Kanals 44.

Die in der Flüssigkeit in dem ersten Kanal 45 vorhandenen Ionen unterliegen durch die Strömung senkrecht zum Magnetfeld 43 einer Ladungstrennung. Diese kann als Potentialdifferenz an jeweils an dem ersten und dem rückführenden Abschnitt des ersten Kanals angeordneten ersten 403 und weiterem 402 Elektrodenpaar abgeleitet werden.

Ebenso führt die Strömung im zweiten Kanal 44 zu einer Ladungstrennung, die als Potentialdifferenz an jeweils an dem ersten und dem rückführenden Abschnitt 42 des zweiten Kanals angeordneten zweiten 405 und vierten 401 Elektrodenpaar abgeleitet werden kann.

Das erste Elektrodenpaar 403, das zweite Elektrodenpaar 405, das weitere Elektrodenpaar 402, sowie das vierte Elektrodenpaar 401 sind über Messleitungen 420 mit der Auswerteeinheit 404 verbunden.

Die Auswerteeinheit 440 ist konfiguriert, um einerseits eine Flüssigkeitsbilanz zwischen dem Volumenstrom im ersten 45 und im zweiten 44 fluidführenden Kanal zu bestimmen und andererseits eine Änderung der Messbedingungen, insbesondere eine Lageänderung des Kassettenmoduls 46 im Magnetfeld 43 festzustellen.

Die Bestimmmung der Flüssigkeitsbilanz oder des Differenzflusses zwischen dem ersten 45 und dem zweiten 44 fluidführenden Kanal erfolgt auf Grund eines Vergleichs der an dem ersten Elektrodenpaar 403 abgeleiteten Spannung und der am zweiten Elektrodenpaar 405 abgeleiteten Spannung in der Auswerteeinheit 404.

Die Flüssigkeitsdifferenz zwischen dem ersten und dem zweiten fluidführenden Kanal kann als Differenz zwischen dem Volumenstrom im ersten fluidführenden Kanal 45 und dem im zweiten fluidführenden Kanal 44 angegeben werden.

Wenn durch Ansteuern einer Kalibrationsvorrichtung gleiche Volumenströme in ersten Kanal 45 und im zweiten 44 fluidführenden Kanal vorherrschen, so kann durch Vergleich der am ersten Elektrodenpaar 403 abgeleiteten Spannung mit der am zweiten Elektrodenpaar 405 abgeleiteten Spannung ein Nullpunkt der Flüssigkeitsbilanz bestimmt und als Kalibrationswert hinterlegt werden. Eine entsprechende Kalibrationsvorrichtung ist im Zusammenhang mit den Figuren 6 a und 6 b beschrieben.

Die Bestimmung der Änderung der Messbedingungen oder der Lageänderung erfolgt durch Vergleich der Spannungen an den an demselben Kanal angeordneten Elektrodenpaare, d.h. durch Vergleich der am ersten 403 und am weiteren 402 Elektrodenpaar Elektrodenpaar abgeleiteten Spannung und/oder durch Vergleich der am zweiten 405 und am vierten 401 Elektrodenpaar abgeleiteten Spannung.

Wird eine Änderung der Messbedingungen festgestellt, so wird vorteilhaft eine Kalibrierung initiiert und oben beschrieben durchgeführt.

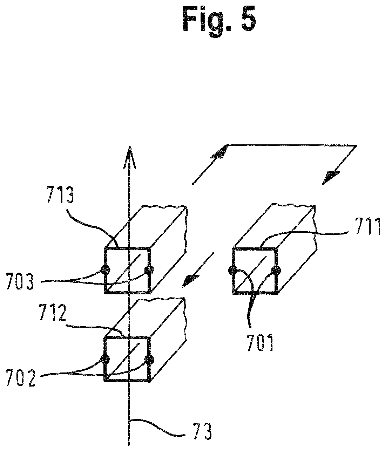

Figur 5 zeigt eine räumliche Darstellung einer Anordnung von fluidführenden Kanälen 712 und deren Abschnitten 713, 711 eines Kassettenmoduls in Bezug auf ein Magnetfeld 73 in einem elektromagnetischen Differenzflussmesser. Hinsichtlich der Funktion entspricht der Differenzflussmesser im Wesentlichen dem im Zusammenhang mit der Figur 4a beschriebenen Differenzflussmesser, auf diese Beschreibung wird Bezug genommen an Stelle einer Wiederholung. Dargestellt sind der erste Abschnitt 713 des ersten Kanals, der rückführende Abschnitt 711 des ersten Kanals, sowie der zweite Kanal 712, die jeweils in dem Kassettenmodul ausgebildet sind. Die in Figur 5 dargestellte Anordnung der im Betrieb fluidführenden Kanäle beziehungsweise deren Abschnitte 713, 712 und 711 in Bezug auf die Richtung des Magnetfelds 73 unterscheidet sich von der Anordnung der Figur 4a. In der Ausführungsform der Figur 5 sind der erste Abschnitt 713 des ersten Kanals und der zweite Kanal 712 in Bezug auf die Richtung des Magnetfelds hintereinander angeordnet. Bei dieser Anordnung ist nur eine geringe Empfindlichkeit des Differenzflusssensors gegeben, wenn sich die Lage der fluidführenden Kanäle 712, 713, 711 im Magnetfeld ändert. Andererseits sind der erste 713 und der rückführende 711 Abschnitt des ersten Kanals in Bezug auf die Richtung des Magnetfelds 73 nebeneinander angeordnet. Wenn eine Inhomogenität oder Asymmetrie des Magnetfelds gegeben ist, kann so Änderung der Lage des Kassettenmoduls im Magnetfeld mit einer großen Sensitivität erkannt werden.

Der erste Abschnitt 713 des ersten fluidführenden Kanals wird in einer Strömungsrichtung im Wesentlichen entgegengesetzt zur Strömungsrichtung im zweiten fluidführenden Kanal 711 durchströmt.

Ebenso werden der erste 713 und der rückführende Abschnitt 711 des ersten Kanals in im Wesentlichen entgegengesetzten Strömungsrichtungen durchströmt. Elektrodenpaare 703, 701, sind an dem ersten 713 und am zweiten 711 Abschnitt des ersten fluidführenden Kanals angeordnet, und ein Elektrodenpaar 702 ist an dem zweiten fluidführenden Kanal angeordnet. Die Verbindungslinien zwischen den Elektroden der Elektrodenpaare 701, 702, 703 sind jeweils im Wesentlichen senkrecht zur Richtung des Magnetfelds 73, sowie zur Strömung in den fluidführenden Kanälen angeordnet.

Fig. 6a zeigt eine Ausgestaltung eines Differenzflussmessers 601 mit einer Kalibriereinheit 602. Der Differenzflussmesser 601 ist vorteilhaft entsprechend dem im Zusammenhang mit der Figur 4a beschriebenen Differenzflussmesser 305 oder entsprechend dem im Zusammenhang mit der Figur 4b beschriebenen Differenzflussmesser 415 ausgestaltet. Der Differenzflussmesser 601 verfügt über eine Auswerte- und Steuerungseinheit 64, die hinsichtlich der Funktion als Auswerteeinheit die Funktionen der in der Figur 4 a beschriebenen Auswerteeinheit 304 und/oder der in der Figur 4b beschriebenen Auswerteeinheit 404 ausführt.

Dargestellt sind weiterhin ein Zufluss 65 zu dem ersten Kanal des Differenzflussmessers 601 und ein Abfluss 67 von dem ersten Kanal des Differenzflussmessers, sowie ein Zufluss 68 von dem zweiten Kanal und ein Abfluss 66 von dem zweiten Kanal.

Zwischen dem Abfluss 67 und dem Zufluss 68 ist eine Kurzschlussleitung 69 mit einem Absperrorgan 61 angeordnet. Weiterhin sind Absperrorgane 62 und 63 in dem Abfluss 67 sowie in dem Zufluss 68 angeordnet.

Die Steuereinheit 64 ist über Steuerleitungen 66 mit den Absperrorganen 61, 62, 63 verbunden.

Für den Differenzflussmesser 601 sind grundsätzlich zwei Betriebsarten vorgesehen: eine Betriebsart 'Bilanzieren', und eine Betriebsart 'Kalibrieren'. In der Betriebsart 'Bilanzieren' ist das Absperrorgan 61 der Kurzschlussleitung 69 geschlossen oder geschlossen gehalten und der Differenzflussmesser bestimmt eine Flüssigkeitsbilanz, wie oben im Zusammenhang mit dem Differenzflussmesser 305 der Figur 4 a oder dem Differenzflussmesser 415 der Figur 4b beschrieben. Die geschlossene Stellung des Absperrorgans 61 in der Betriebsart 'Bilanzieren' ist in der Figur 6a durch die durchgefärbte Darstellung des Absperrorgans 61 dargestellt.

In der Betriebsart 'Bilanzieren' sind die Absperrorgane 62 und 63 geöffnet oder offen gehalten, was in der Figur 6a durch die transparente Darstellung der Absperrorgane 62 und 63 angedeutet ist.

In der Betriebsart 'Kalibrieren' wird eine Kalibration des Differenzflussmessers, insbesondere der Auswerte- und Kontrolleinheit 64 vorgenommen. Die Kalibration der Auswerte- und Kontrolleinheit dient dem Bestimmen eines Nullpunkts der Flüssigkeitsbilanz, d.h. eines Betriebspunktes, in dem der Flüssigkeitsfluss im ersten Kanal und der Flüssigkeitsfluss im zweiten Kanal übereinstimmen, wie oben im Zusammenhang mit der Figur 4a und der Figur 4b beschrieben.

In der Betriebsart 'Kalibrieren' steuert die Auswerte- und Steuereinheit die Absperrorgane 61, 62, 63 an, so dass eine Kurzschluss zwischen dem Abfluss 67 und dem Zufluss 68, und somit ein Kurzschluss zwischen dem ersten und dem zweiten Kanal hergestellt wird. Dazu wird das Absperrorgan 61 in der Kurzschlussleitung 69 geöffnet oder offen gehalten und die Absperrorgane 62 und 63 werden geschlossen oder geschlossen gehalten.

Die offene Stellung des Absperrorgans 61 in der Kurzschlussleitung 69 ist in der Figur 6b durch die transparente Darstellung des Absperrorgans 61 angedeutet. Die geschlossene Stellung der Absperrorgane 62, 63 ist in der Figur 6b durch die durchgefärbte Stellung dargestellt.

## Patentansprüche

1. Kassettenmodul (32, 46) für einen Differenzflussmesser (305, 415), wobei das Kassettenmodul (32, 46) einen ersten (30, 45) und einen zweiten (31, 44) im Betrieb des Differenzflussmessers fluidführenden Kanal ausbildet, die im Betrieb des Differenzflussmessers (305, 415) jeweils von einem Magnetfeld (33, 43) durchdrungen sind, mit jeweils einem an dem ersten (30, 45) und an dem zweiten (31, 44) Kanal angeordneten Elektrodenpaar (301, 302, 403, 405) so dass durch einen Vergleich des Signals am ersten (301, 403) und am zweiten (302, 405) Elektrodenpaar eine Flussdifferenz zwischen dem ersten (30, 44) und dem zweiten (31, 45) fluidführenden Kanal bestimmbar ist, **dadurch gekennzeichnet, dass** der erste (30, 45) Kanal einen im Betrieb von dem Magnetfeld durchdrungenen weiteren Abschnitt (34, 41), vorzugsweise einen rückführenden Abschnitt (34, 41) ausbildet, ein weiteres Elektrodenpaar (303, 402) in dem weiteren Abschnitt (34, 41) angeordnet ist, so dass durch Vergleich des Signals an dem ersten (301, 403) und an dem weiteren (303, 402) Elektrodenpaar eine Änderung der Messbedingungen feststellbar ist.

2. Kassettenmodul (46) für einen Differenzflussmesser (415) nach Anspruch 1, wobei der zweite im Betrieb fluidführende Kanal (44) einen im Betrieb von dem Magnetfeld durchdrungenen weiteren Abschnitt (42), vorzugsweise einen rückführenden Abschnitt ausbildet, ein viertes Elektrodenpaar (401) in dem weiteren Abschnitt (42) angeordnet ist, so dass durch Vergleich der Spannung an dem zweiten (405) und an dem vierten (401) Elektrodenpaar eine Änderung der Messbedingungen feststellbar ist.

3. Differenzflussmesser (305, 415) mit einem Kassettenmodul (32, 46) nach Anspruch 1, und mit einer mit dem ersten (301, 405), dem zweiten (302, 403) und dem weiteren (303, 402) Elektrodenpaar verbundenen Auswerteeinheit (304, 404), wobei die Auswerteeinheit (304, 404) so konfiguriert ist, dass durch einen Vergleich des Signals am ersten (301, 403) und am zweiten (302, 405) Elektrodenpaar eine Flussdifferenz zwischen dem ersten (30, 45) und dem zweiten (31, 44) fluidführenden Kanal bestimmbar ist, und wobei die Auswerteeinheit (304, 404) weiterhin so konfiguriert ist, dass durch einen Vergleich des Signals an dem ersten (301, 403) und an dem weiteren (303, 402) Elektrodenpaar eine Änderung der Messbedingungen feststellbar ist.

4. Differenzflussmesser (305, 415) nach Anspruch 3, wobei die Auswerteeinheit (304, 404) so konfiguriert ist, dass durch einen Vergleich des Signals an dem ersten (301, 403) und an dem weiteren (303, 402) Elektrodenpaar eine Lageänderung des Kassettenmoduls im Magnetfeld (33, 43) feststellbar ist.

5. Differenzflussmesser (415) nach einem der Ansprüche 3 oder 4, mit einem Kassettenmodul nach Anspruch 2, wobei die Auswerteeinheit (404) so konfiguriert ist, dass durch einen Vergleich des Signals an dem zweiten (405) und an dem vierten (401) Elektrodenpaar eine Änderung der Messbedingungen feststellbar ist.

6. Differenzflussmesser (415) nach Anspruch 5, wobei die Auswerteeinheit (404) so konfiguriert ist, dass durch einen Vergleich des Signals an dem zweiten (405) und dem vierten (401) Elektrodenpaar eine Lageänderung des Kassettenmoduls (46) im Magnetfeld (43) feststellbar ist.

7. Differenzflussmesser (415) nach einem der Ansprüche 5 oder 6, wobei der zweite fluidführende Kanal (44) einen ersten und einen rückführenden (42) Abschnitt aufweist und wobei der erste und der rückführende (42) Abschnitt in Bezug auf die Richtung des Magnetfelds (43) nebeneinander angeordnet sind.

8. Differenzflussmesser (305) nach einem der Ansprüche 3 bis 7, wobei der erste (713) und der zweite (712) fluidführende Kanal in Bezug auf die Richtung des Magnetfelds (73) hintereinander angeordnet sind.

9. Differenzflussmesser (305) nach einem der Ansprüche 3 - 8, wobei der erste fluidführende Kanal einen ersten (713) und einen rückführenden (711) Abschnitt aufweist und wobei der erste (713) und der rückführende (711) Abschnitt in Bezug auf die Richtung des Magnetfelds (73) nebeneinander angeordnet sind.

10. Differenzflussmesser nach einem der Ansprüche 3 - 9, wobei die Auswerteeinheit angepasst ist, als Antwort auf eine Feststellung der Änderung der Messbedingungen eine Rekalibrierung zu initiieren,

11. Differenzflussmesser nach Anspruch 10, angepasst, als Antwort auf die Feststellung der Änderung der Messbedingungen eine Rekalibrierung durchzuführen.

12. Differenzflussmesser nach Anspruch 11 mit einer Kurzschlussleitung (69) und Absperrorganen (61, 62, 63) zum Kurzschließen des ersten und des zweiten Kanals, und wobei zur Rekalibrierung die Absperrorgane derart ansteuerbar sind, dass der erste und der zweite Kanal kurzgeschlossen werden.

13. Differenzflussmesser nach Anspruch 12, wobei jeweils ein Absperrorgan in einem Zufluss (63), in einem Abfluss (62) sowie in der Kurzschlussleitung (61) angeordnet ist.

## Claims

1. A cassette module (32, 46) for a differential flowmeter (305, 415), wherein the cassette module (32, 46) forms a first fluid-carrying channel (30, 45) and a second fluid-carrying channel (31, 44) during operation of the differential flowmeter, these channels each being permeated by a magnetic field (33, 43) during operation of the differential flowmeter (305, 415), each having an electrode pair (301, 302, 403, 405) arranged on the first channel (30, 45) and on the second channel (31, 44), so that a flow difference between the first fluid-carrying channel (30, 44) and the second fluid-carrying channel (31, 45) can be determined by comparing the signal on the first electrode pair (301, 403) and on the second electrode pair (302, 405), **characterized in that** the first channel (30, 45) forms an additional section (34, 41) permeated by the magnetic field during operation, preferably a return section (34, 41), another electrode pair (303, 402) being arranged in the additional section (34, 41), so that a change in the measurement conditions can be ascertained by comparing the signal on the first electrode pair (301, 403) and on the additional electrode pair (303, 402).

2. The cassette module (46) for a differential flowmeter (415) according to claim 1, wherein the second channel (44) which carries fluid during operation forms an additional section (42), preferably a return section which is permeated by the magnetic field during operation, a fourth electrode pair (401) is arranged in the additional section (42), so that a change in the measurement conditions can be detected by comparing the voltage on the second electrode pair (405) and on the fourth electrode pair (401).

3. A differential flowmeter (305, 415) having a cassette module (32, 46) according to claim 1 and having an evaluation unit (304, 404), which is connected to the first electrode pair (301, 405) and to the second electrode pair (302, 403) and the additional electrode pair (303, 402), wherein the evaluation unit (304, 404) is configured so that a difference in flow between the first fluid-carrying channel (30, 45) and the second fluid-carrying channel (31, 44) can be detected by comparing the signal on the first electrode pair (301, 403) and the signal on the second electrode pair (302, 405), and wherein the evaluation unit (304, 404) is additionally configured so that a change in the measurement conditions can be detected by comparing the signal on the first electrode pair (301, 403) and on the additional electrode pair (303, 402).

4. The differential flowmeter (305, 415) according to claim 3, wherein the evaluation unit (304, 404) is configured so that a change in position of the cassette module in the magnetic field (33, 43) can be detected by comparing the signal on the first electrode pair (301, 403) with the signal on the additional electrode pair (303, 402).

5. The differential flowmeter (415) according to any one of claims 3 or 4, having a cassette module according to claim 2, wherein the evaluation unit (404) is configured so that a change in the measurement conditions can be detected by comparing the signal on the second electrode pair (405) and the signal on the fourth electrode paid (401) .

6. The differential flowmeter (415) according to claim 5, wherein the evaluation unit (404) is configured so that a change in position of the cassette module (46) in the magnetic field (43) can be detected by comparing the signal on the second electrode pair (405) with the signal on the fourth electrode pair (401).

7. The differential flowmeter (415) according to any one of claims 5 or 6, wherein the second fluid-carrying channel (44) has a first section and a return section (42), and wherein the first section and the return section (42) are arranged side by side with respect to the direction of the magnetic field (43).

8. The differential flowmeter (305) according to any one of claims 3 to 7, wherein the first fluid-carrying channel (713) and the second fluid-carrying channel (712) are arranged one after the other with respect to the direction of the magnetic field (73).

9. The differential flowmeter (305) according to any one of claims 3 to 8, wherein the first fluid-carrying channel has a first section (713) and a return section (711), and wherein the first section (713) and the return section (711) are arranged side by side with respect to the direction of the magnetic field (73).

10. The differential flowmeter according to any one of claims 3 to 9, wherein the evaluation unit is adjusted to initiate a recalibration in response to detection of the change in the measurement conditions.

11. The differential flowmeter according to claim 10, adjusted to carry out a recalibration in response to detection of the change in the measurement conditions.

12. The differential flowmeter according to claim 11, having a short circuit line (69) and cutoff elements (61, 62, 63) for short-circuiting the first and second channels, and wherein the cutoff elements are controllable for recalibration, so that the first and second channels are shortcircuited.

13. The differential flowmeter according to claim 12, wherein a cutoff element is arranged in an inlet (63), in an outlet (62) and in the short-circuit line (61).

## Revendications

1. Module de cassette (32, 46) pour débitmètre différentiel (305, 415), le module de cassette (32, 46) constituant en cours de fonctionnement du débitmètre différentiel un premier (30, 45) et un second (31, 44) canal conducteur de fluide qui sont traversés respectivement en cours de fonctionnement du débitmètre différentiel (305, 415) par un champ magnétique (33, 43), comportant respectivement une paire d'électrodes (301, 302, 403, 405) disposées au niveau du premier (30, 45) et du second (31, 44) canal, de sorte que, par comparaison du signal au niveau de la première (301, 403) et de la deuxième (302, 405) paire d'électrodes, une différence d'écoulement entre le premier (30, 45) et le second (31, 44) canal conducteur de fluide est définissable, **caractérisé en ce que** le premier (30, 45) canal constitue une autre section (34, 41), de préférence une section de renvoi (34, 41), traversée en cours de fonctionnement par le champ magnétique, qu'une autre paire d'électrodes (303, 402) est disposée dans l'autre section (34, 41), de sorte que, par comparaison du signal au niveau de la première (301, 403) et de la deuxième (302, 405) paire d'électrodes, une modification des conditions de mesure est constatable.

2. Module de cassette (46) pour débitmètre différentiel (415) selon la revendication 1, dans lequel le second canal (44) conducteur de fluide constitue en cours de fonctionnement une autre section (42), de préférence une section de renvoi, traversée en cours de fonctionnement par le champ magnétique, une quatrième paire d'électrodes (401) est disposée dans l'autre section (42), de sorte que, par comparaison de la tension au niveau de la deuxième (405) et de la quatrième (401) paire d'électrodes, une modification des conditions de mesure est constatable.

3. Débitmètre différentiel (305, 415) comportant un module de cassette (32, 46) selon la revendication 1, et une unité d'exploitation (304, 404) connectée à la première (301, 405), la deuxième (302, 403) et à l'autre (303, 402) paire d'électrodes, l'unité d'exploitation (304, 404) étant conçue de manière à ce que, par comparaison du signal au niveau de la première (301, 405) et la deuxième (302, 403) paire d'électrodes, une différence de flux entre le premier (30, 45) et le second (31, 44) canal conducteur de fluide soit définissable, l'unité d'exploitation (304, 404) étant en outre conçue de manière à ce que, par comparaison du signal au niveau de la première (301, 403) et de l'autre (303, 402) paire d'électrodes, une modification des conditions de mesure soit constatable.

4. Débitmètre différentiel (305, 415) selon la revendication 3, dans lequel l'unité d'exploitation (304, 404) est conçue de manière à ce que, par comparaison du signal au niveau de la première (301, 405) et de l'autre (303, 402) paire d'électrodes, une modification de la position du module de cassette dans le champ magnétique (33, 43) soit constatable.

5. Débitmètre différentiel (415) selon une des revendications 3 ou 4, comportant un module de cassette selon la revendication 2, dans lequel l'unité d'exploitation (404) est conçue de manière à ce que, par comparaison du signal au niveau de la deuxième (405) et de la quatrième (401) paire d'électrodes, une modification des conditions de mesure soit constatable.

6. Débitmètre différentiel (415) selon la revendication 5, dans lequel l'unité d'exploitation (404) est conçue de manière à ce que, par comparaison du signal au niveau de la deuxième (405) et de la quatrième (401) paire d'électrodes, une modification de la position du module de cassette (46) dans le champ magnétique (43) soit constatable.

7. Débitmètre différentiel (415) selon une des revendications 5 ou 6, dans lequel le second canal conducteur de fluide (44) présente une première section et une section de renvoi (42) et dans lequel la première section et la section de renvoi (42) sont juxtaposées par rapport au sens du champ magnétique (43).

8. Débitmètre différentiel (305) selon une des revendications 3 à 7, dans lequel le premier (713) et le second (712) canal conducteur de fluide sont disposés successivement par rapport au sens du champ magnétique (73).

9. Débitmètre différentiel (305) selon une des revendications 3 à 8, dans lequel le premier canal conducteur de fluide présente une première section (713) et une section de renvoi (711), et la première section (713) et la section de renvoi (711) sont juxtaposées par rapport au sens du champ magnétique (73) .

10. Débitmètre différentiel (415) selon une des revendications 3 à 9, dans lequel l'unité d'exploitation est apte à initier un réétalonnage en réponse à une constatation de la modification des conditions de mesure.

11. Débitmètre différentiel selon la revendication 10, qui est apte à réaliser un réétalonnage en réponse à une constatation de la modification des conditions de mesure.

12. Débitmètre différentiel selon la revendication 11, comportant une conduite de court-circuit (69) et des organes de blocage (61, 62, 63) pour court-circuiter le premier et le second canal et, pour le réétalonnage, les organes de blocage sont contrôlables de manière à ce que le premier et le second canal soient court-circuités.

13. Débitmètre différentiel selon la revendication 12, dans lequel un organe de blocage est disposé respectivement dans une conduite d'afflux (63), dans une conduite de reflux (62) et dans la conduite de court-circuit (61).
